# EUROPEAN PATENT APPLICATION

(11) **EP 4 331 627 A1**
(43) Date of publication of application: **06.03.2024**
(21) Application number: 22193453.2
(22) Date of filing: 01.09.2022
(51) Int. Cl.: A61L 2/10

(54) **UV BOOTH**

(71) Applicant: UV Medico A/S, 8230 Åbyhøj (DK)
(72) Inventor: JOHANSEN, Peter, 8230 Åbyhøj (DK); TØNNING, Peter, 8230 Åbyhøj (DK); BANDHOLM, Mikkel, 8230 Åbyhøj (DK); RYTTER, Rakel, 8230 Åbyhøj (DK)
(74) Representative: Zacco Denmark A/S

(57) **Abstract**

A UV booth (1) for decontaminating gowned workers (6) is disclosed, which UV booth (1) comprises at least two walls (2), each of which are equipped with one or more far UVC light sources (3) arranged to emit far UVC light with wavelengths in the range 190-230 nm in one or more directions at least partly towards the centre of the UV booth (1) and/or towards one or more other walls (2) of the UV booth (1). Furthermore, the use of such a UV booth (1) is disclosed.

## Description

The present invention relates to a UV Booth using far UVC light to decontaminate workers gowned in personal protective equipment.

### Background of the invention

At a variety of facilities, it is of uttermost importance to prevent viruses and bacteria from contaminating different highly controlled environments, such as cleanrooms, operation rooms and laboratories, or at least to keep such contamination to an absolute minimum level. For that reason, workers entering such environments are requested to wash/bathe and to change clothes, before gowning themselves in personal protective equipment, such as for instance specialized gowns, facemasks, googles and gloves. Such procedures significantly reduces the risk of contamination by viruses and bacteria entering the controlled environment with the workers. However, the simple fact that the workers touch the protective equipment when putting it on, means that a certain - although usually low - amount of viruses and bacteria will always be present on the outside of the protective equipment, when the workers have finished the procedure and are ready to enter the controlled environment.

### Brief description of the invention

It is a purpose of the present invention to provide an additional line of defence to the known prevention of contamination of highly controlled environments as is described above. As will be described in the following, an effective, touch-free, chemical-free and quick decontamination procedure can be obtained using the present invention.

The present invention relates to a UV booth for decontaminating gowned workers, which UV booth comprises at least two walls, each of which are equipped with one or more far UVC light sources arranged to emit far UVC light with wavelengths in the range 190-230 nm in one or more directions at least partly towards the centre of the UV booth and/or towards one or more other walls of the UV booth.

The ultraviolet C-band (UVC) covers the wavelength range 100-280 nm. UVC light has proven effective for sterilizing air, liquids and surfaces. Furthermore, it has been established in recent years that light in the wavelength range 190-230 nm, called far UVC, is heavily absorbed in the outer layers of the skin and the eye and, hence, is harmless to mammals, including humans, at moderate intensities like the ones used in conventional UVC disinfection.. This means that light in the far UVC spectral region is particularly useful for germicidal purposes in the presence of humans and animals.

Tests with far UVC light and more conventional UVC light with longer wavelengths, such as for instance 254 nm, on materials, from which personal protective equipment, such as gowns, googles, facemasks and gloves, are normally produced, have shown that
- the transmission of far UVC light through such materials is negligible, whereas a significant part of the conventional UVC light is transmitted through such materials
- the degradation of such materials caused by far UVC light is minimal compared to the degradation caused by UVC light with longer wavelengths resulting in a longer lifetime of the personal protective equipment if far UVC light is used

Thus, not only is far UVC light far less harmful to human beings than conventional UVC light. The amount of UV light reaching the living cells of the body through the personal protective equipment and the dead skin cells forming the outer layer of human skin is much smaller with far UVC light than with conventional UVC light.

This allows for the use of far UVC light at high intensity for decontamination of gowned workers, whereby the air and the surfaces can be decontaminated in a short time. Compared to the light intensities of excimer lamps known in the art to be used for disinfection of air and surfaces in densely populated premises where there is a risk of passing on infection, the light intensities used with the present invention may be 50-100 times higher. It should be noted that the purpose of the present invention is not to prevent the passing on of infection but rather to obtain further cleanliness of already clinically clean highly specialized personnel.

The international standard ISO 15858 specifies minimum human safety requirements for the use of UVC lamp devices. In particular, it describes threshold values for UVC exposure levels under which most people can work consistently for eight hours a day, day after day, without adverse effects. For instance, it defines that the radiant exposure from far UVC light with a wavelength of 222 nm should be less than 23 mJ/cm². These levels being defined for direct exposure of human skin and eyes, the standard requires that personal protective equipment must be worn, should the exposure exceed the defined levels. This personal protective equipment is defined to consist of UV resistant eyewear, such as googles, face shields and safety glasses, and of clothing known to be non-transparent to UVC penetration, which covers exposed skin. Hence, through validation that no significant far UVC penetration of the personal protective equipment used at the facility takes place, an increased dosage of far UVC can be administered to the gowned personnel. Also, it should be noted that the defined safety levels are calculated for consistent exposure to UVC light, whereas the decontamination taking place in the UV booth only takes a short time.

The dosage of far UVC light needed depends on the types of viruses and bacteria to be killed. The literature within the art contains information about the amount of light needed to kill, for instance, 99% of a given bacteria using far UVC light with a wavelength of 222 nm. This kind of information is used for dimensioning the far UVC lamps, for choosing the number of far UVC lamps and for setting the duration of a decontamination cycle in the UV booth.

In an embodiment of the invention, the height and the width of the walls are dimensioned so that a worker being decontaminated in the UV booth can be fully accommodated within a three-dimensional space being delimited by the walls.

Dimensioning the UV booth to fully accommodate the gowned worker makes it possible to complete the decontamination process in only one decontamination cycle.

The three-dimensional space being delimited by the walls is to be understood as the space whose edges are defined by the edges of the walls, although not all sides of the space are necessarily covered by a wall. Thus, for instance, the UV booth may comprise two parallel walls constituting opposing sides of a rectangular space. In this way, the two walls of the UV booth form a portal, through which the gowned worked can pass for decontamination of his/her personal protective equipment. In another embodiment of the invention, the UV booth may comprise three walls constituting three sides of a rectangular space. In this case, the gowned worker will enter the UV booth through the open side of the rectangular space and leave it again through the same opening after decontamination of the personal protective equipment.

In an embodiment of the invention, the directions, in which the far UVC light sources emit the far UVC light, are substantially horizontal and perpendicular to the walls, from which the far UVC light is emitted.

In an embodiment of the invention, the far UVC light sources are distributed evenly across the surfaces of the walls facing towards the centre of the UV booth, for instance by being arranged in arrays of one or more horizontal rows and one or more vertical columns.

In this way, it is relatively simple to ensure an even density of the far UVC light and an even coverage of the gowned worker being decontaminated.

In an embodiment of the invention, the UV booth further comprises a plurality of far UVC light sources arranged along the bottom of one or more of the walls to emit far UVC light in one or more partly upward directions towards the centre of the UV booth.

In this way, it is possible also to treat partly downward-facing surfaces of the gowned worker, which are less accessible for the substantially horizontal far UVC light.

In an embodiment of the invention, the UV booth further comprises a plurality of far UVC light sources arranged along the top of one or more of the walls to emit far UVC light in one or more partly downward directions towards the centre of the UV booth.

In this way, it is possible also to treat partly upward-facing surfaces of the gowned worker, which are less accessible for the substantially horizontal far UVC light.

In an embodiment of the invention, the UV booth further comprises a plurality of far UVC light sources arranged along a vertical edge of one or more of the walls to emit far UVC light in one or more directions towards the centre of the UV booth, which directions are substantially horizontal but not perpendicular to the walls, from which the far UVC light is emitted.

The use of far UVC light sources, which are arranged at a certain angle with respect to the wall makes it possible also to treat surfaces of the gowned worker, which are not facing a wall equipped with far UVC light sources. Thus, this feature is especially useful along those vertical edges of the walls, which abut an open side of the UV booth.

In an embodiment of the invention, the number of far UVC light sources is larger than 20, preferably larger than 50, most preferably larger than 100.

A relatively high number of far UVC light sources are needed to obtain an even distribution of far UVC light within the UV booth.

In an embodiment of the invention, one or more sides of the three-dimensional space delimited by the walls, which are not covered by a wall, are provided with doors, such as glass doors.

In an embodiment of the invention, the doors are coated with a UVC-reflecting material on the side facing towards the centre of the UV booth when the doors are closed.

The use of such doors, especially doors coated with a UVC-reflecting material shields the environment around the UV booth from some of the far UVC light emitted therein. Even more important, the reflection of the far UVC light from the inside of the doors results in a more even distribution of far UVC light unto the gowned worker being decontaminated, also on the side(s) facing the doors.

Some materials, such as for instance some polytetrafluoroethylene (PTFE) materials, are known to reflect more than 95% of far UVC light, at least down to a wavelength of 200 nm.

In an embodiment of the invention, one or more touchless buttons are arranged within the UV booth for allowing the gowned worker to start and stop a decontamination cycle.

The use of touchless buttons ensures a clean and safe operation of the UV booth. For the comfort of the gowned worker being decontaminated, the start and stop of the process are preferably controlled by the gowned worker himself/herself from inside the UV booth. As described below, the contamination cycle normally stops automatically, when the required dosage of far UVC light has been delivered.

In an embodiment of the invention, the wavelengths of the emitted far UVC light is in the range 210-230 nm, preferably in the range 220-225 nm, such as 222 nm.

Far UVC light in these spectral regions has proved very effective for decontamination and at the same time very little harmful to human beings.

In an embodiment of the invention, the UV booth comprises an electronic control system, which is programmed to automatically stop a decontamination cycle, when a predefined dosage of far UVC light has been delivered.

This is the safest way to obtain a sufficient decontamination and at the same time to minimize the amount of far UVC light, to which the gowned worker is exposed.

In an embodiment of the invention, the UV booth is dimensioned, constructed and controlled in such a way that the duration of a contamination cycle is less than two minutes, preferably less than one minute, such as 30 seconds.

Having a short contamination cycle duration reduces the amount of working time being spent on the non-productive, but necessary, decontamination process.

In an aspect of the invention, it relates to the use of a UV booth as described above for decontamination of a gowned worker wearing personal protective equipment, such as a gown, googles, a facemask and/or gloves.

### Figures

A few exemplary embodiments of the invention are described more in detail in the following with reference to the figures, of which
- Fig. 1: illustrates schematically a perspective top view of a first embodiment of a UV booth according to the invention,
- Fig. 2: illustrates schematically a 3D view of the embodiment shown in Fig. 1,
- Fig. 3: illustrates schematically a control panel of a UV booth according to an embodiment of the invention,
- Fig. 4a: illustrates schematically a top view of a second embodiment of a UV booth according to the invention with its doors open,
- Fig. 4b: illustrates schematically a top view of the embodiment shown in Fig. 4a, only with its doors closed,
- Fig. 5a: illustrates schematically a front view of the embodiment shown in Fig. 4a with its door opened, and
- Fig. 5b: illustrates schematically a front view of the embodiment shown in Fig. 4a, only with its doors closed.

### Detailed description of the invention

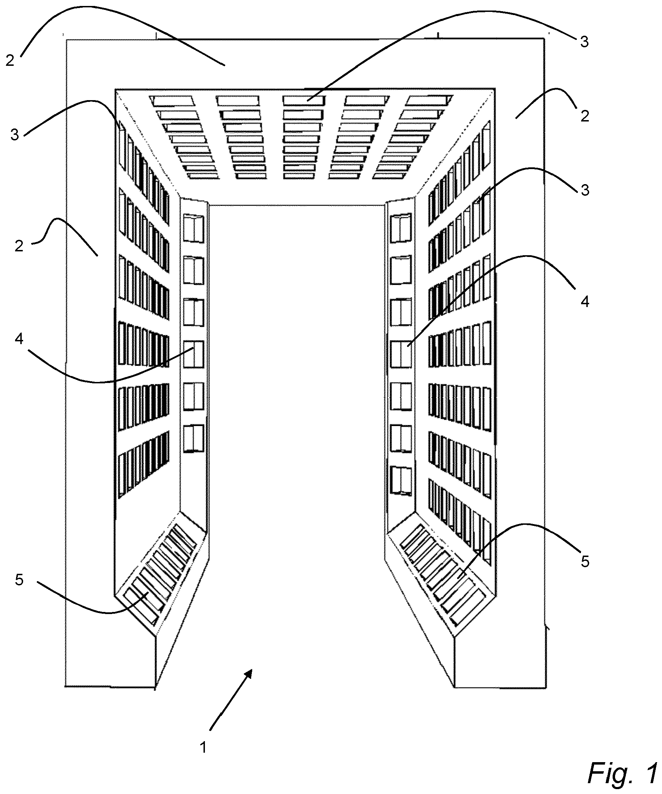

Fig. 1 illustrates schematically a perspective top view of a first embodiment of a UV booth 1 according to the invention. In the illustrated embodiment, the UV booth 1 comprises three walls 2 constituting three sides of a rectangular space. Each of the walls 2 are equipped with eight horizontal rows of far UVC light sources 3 arranged to emit far UVC light in a substantial horizontal direction perpendicular to the wall 2, from which the far UVC light is emitted. Along the bottom of two of the walls 2, a row of upwardly angled far UVC light sources 4 is arranged to treat partly downward-facing surfaces of the gowned worker 6, which are less accessible for the substantially horizontal far UVC light. Also, a vertical column of sideways angled far UVC light sources 5 is arranged along each of the vertical edges of the walls 2 abutting the open side of the rectangular space. These sideways angled far UVC light sources 5 ensures that also the side of the gowned worker 6 (not shown in this figure), which faces the open side of the rectangular space within the UV booth 1, on which there is no wall 2 is exposed to far UVC light.

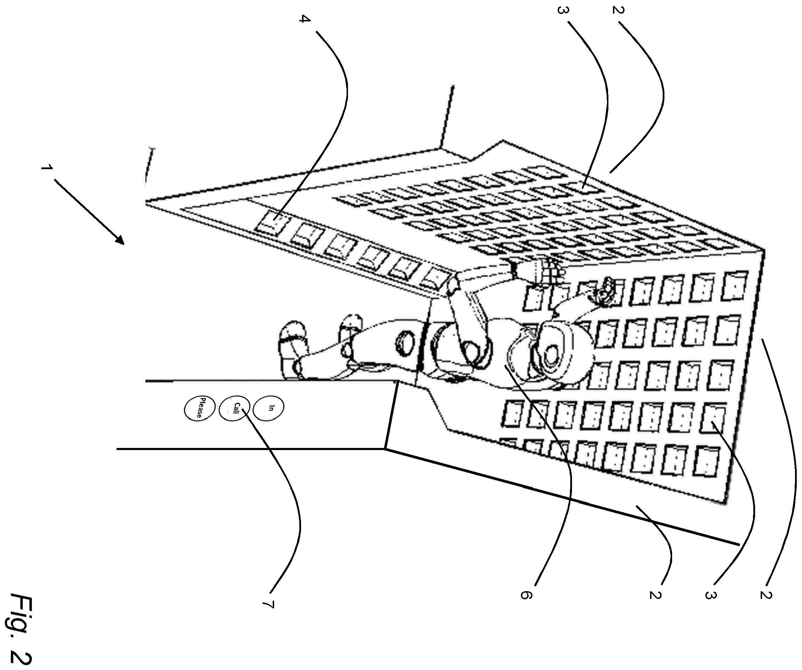

Fig. 2 illustrates schematically a 3D view of the embodiment shown in Fig. 1. Fig. 2 illustrates how the gowned worker 6 poses during the decontamination process with the legs spread and the arms lifted so that all surfaces of the personal protective equipment is exposed to the far UVC light. Also shown in this drawing is an outer display 7 showing persons outside the UV booth 1 the status of the UV booth 1 and signalling when the UV booth is ready for the next gowned worker 6 to be decontaminated.

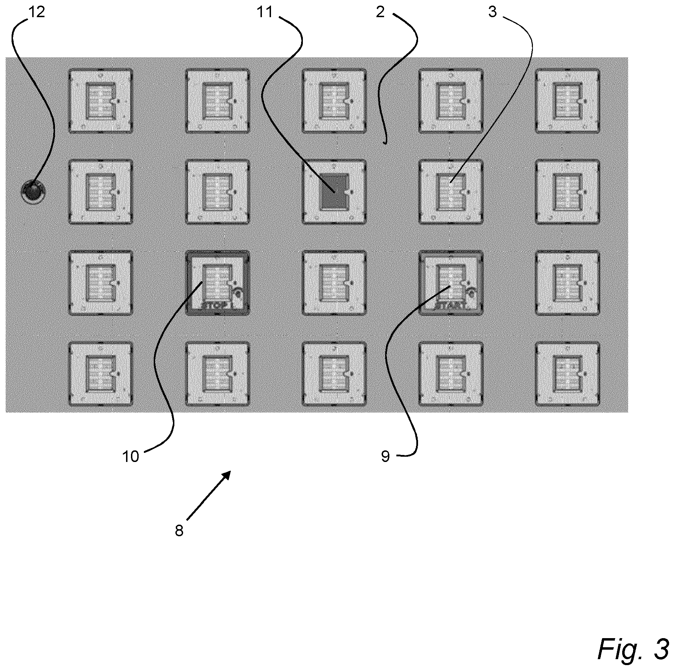

Fig. 3 illustrates schematically a control panel 8 of a UV booth 1 according to an embodiment of the invention. In the illustrated embodiment, the control panel 8 is constituted by a part of one of the walls 2 of the UV booth 1, in which some control features (9-12) are integrated.

A touchless start button 9 is arranged next to one of the far UVC light sources 3 so that the gowned worker 6 (not shown in this figure) being decontaminated can start the decontamination cycle without touching anything. The far UVC light source 3, next to which the touchless start button 9 is arranged, is surrounded by a green light for making it easy for the gowned worker 6 to identify the position of the touchless start button 9.

Similarly, a touchless stop button 10 is arranged next to another one of the far UVC light sources 3 so that the gowned worker 6 being decontaminated can stop the decontamination cycle without touching anything, should he/she wish to do so. It should be noted that, under normal conditions, the UV booth 1 is configured in such a way that the decontamination cycle stops automatically, when the required amount of far UVC light has been delivered. The far UVC light source 3, next to which the touchless stop button 10 is arranged, is surrounded by a red light for making it easy for the gowned worker 6 to identify the position of the touchless stop button 10.

One of the far UVC light sources 3 has been replaced by an inner display 11, which is arranged to visually inform the gowned worker 6 being decontaminated about the status of the decontamination cycle, about the pose to take during the decontamination, etc. It should be noted that, in order to ensure an even exposure of all sides of the gowned worker 6 to the far UVC light, the decontamination process may be designed so that the gowned worker 6 will turn around or in other ways shift posture during the decontamination cycle.

Also, the control panel 8 comprises a mechanical safety stop button 12 arranged to cut the power from the UV booth 1 immediately, when the safety stop button 12 is pushed.

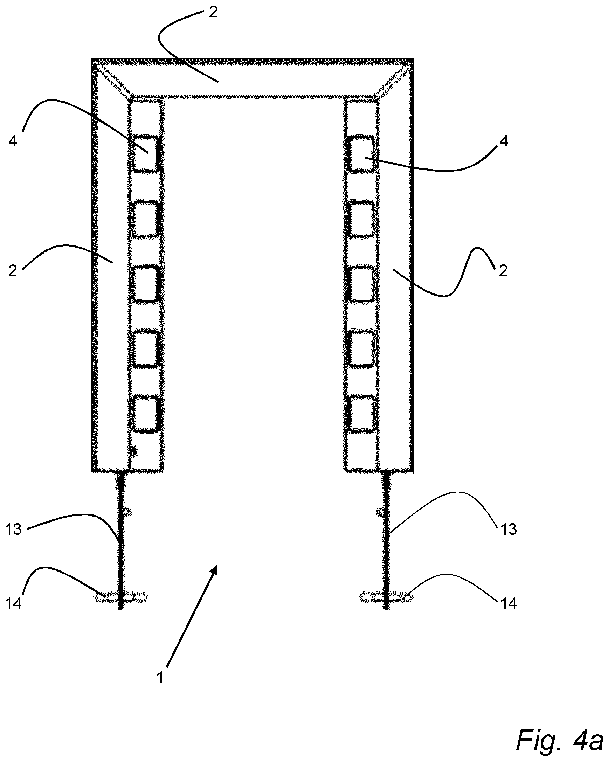

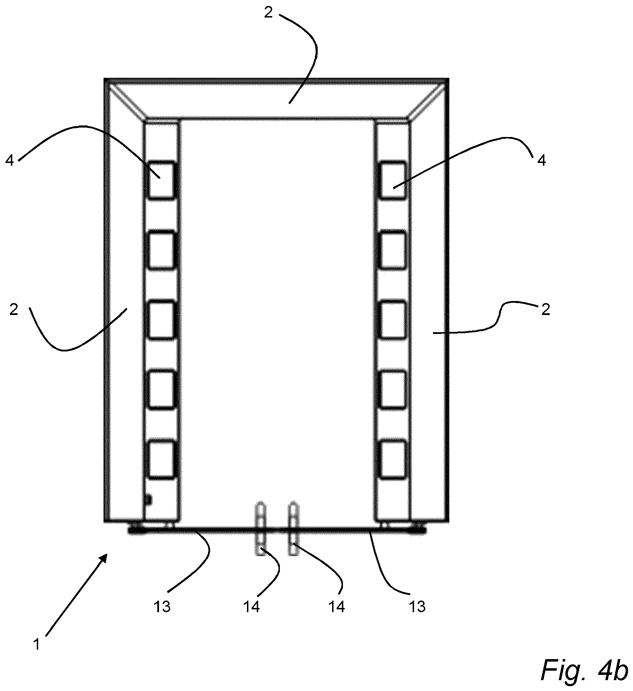

Figs. 4a and 4b illustrate schematically a top view of a second embodiment of a UV booth 1 according to the invention with its doors 13 open and closed, respectively. In this embodiment, there are no sideways angled far UVC light sources 5 as were the case in the embodiment illustrated in Fig. 1 and 2. Instead, the UV booth 1 is equipped with doors 13, which can be opened and closed by means of door handles 14. When the doors 13 are closed as illustrated in Fig. 4b, all four sides of the rectangular space within the UV booth 1 are closed, three of them by a wall 2, the fourth by the doors 13. In order to make sure that the gowned worker 6 (not shown in these figures) is also exposed by far UVC light on the side, which faces the doors 13, i.e. the side, where there are no far UVC light sources 3, the doors 13 are coated on the inside with a UVC-reflecting material (not shown). This ensures that far UVC light emitted from the far UVC light sources 3 in the three walls 2 are reflected from the inside of the closed doors 13 onto the side of the gowned worker 6 facing these doors 13.

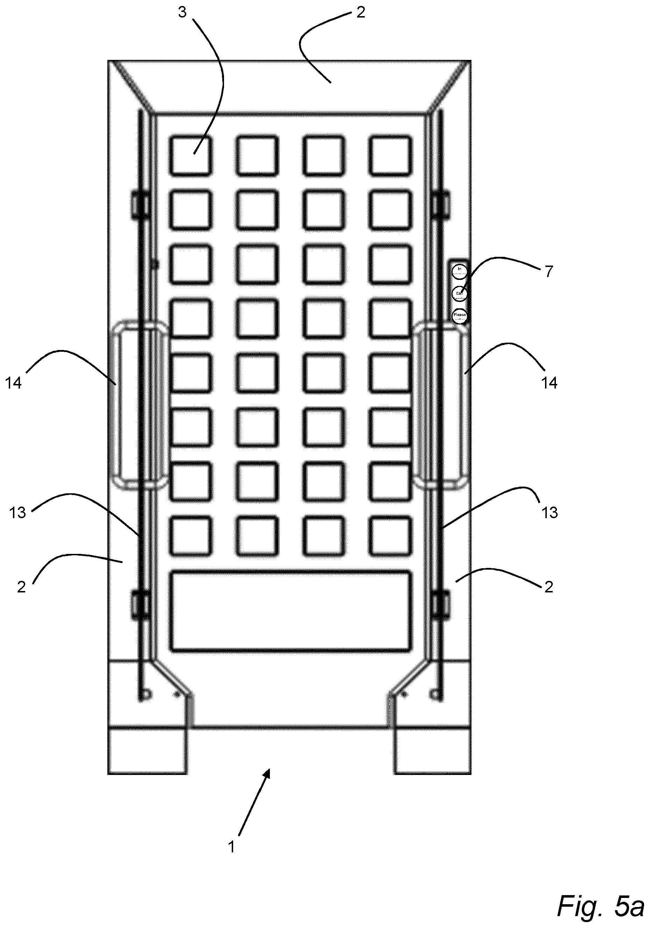

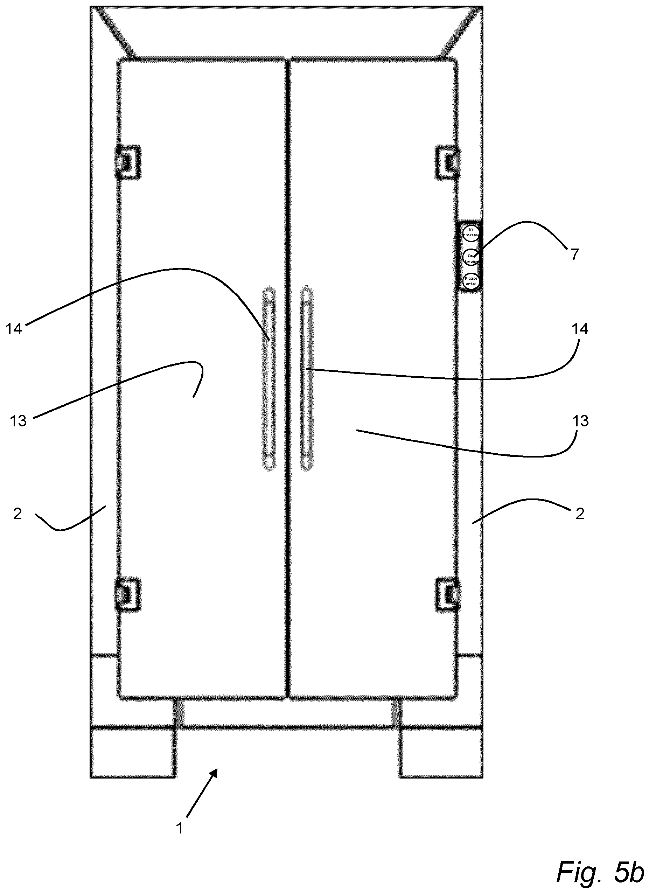

Figs. 5a and 5b illustrate schematically a front view of the embodiment shown in Figs. 4a and 4b with its door opened and closed, respectively.

### List of reference numbers

- 1.: UV booth
- 2.: Wall
- 3.: Far UVC light source in wall
- 4.: Upwardly angled far UVC light source
- 5.: Sideways angled far UVC light source
- 6.: Gowned worker
- 7.: Outer display
- 8.: Control panel
- 9.: Touchless start button
- 10.: Touchless stop button
- 11.: Inner display
- 12.: Mechanical safety stop button
- 13.: Door
- 14.: Door handle

## Claims

1. A UV booth (1) for decontaminating gowned workers (6), which UV booth (1) comprises at least two walls (2), each of which are equipped with one or more far UVC light sources (3) arranged to emit far UVC light with wavelengths in the range 190-230 nm in one or more directions at least partly towards the centre of the UV booth (1) and/or towards one or more other walls (2) of the UV booth (1).

2. The UV booth (1) according to claim 1, wherein the height and the width of the walls (2) are dimensioned so that the gowned worker (6) being decontaminated in the UV booth (1) can be accommodated fully within a three-dimensional space being delimited by the walls (2).

3. The UV booth (1) according to claim 1 or 2, wherein the directions, in which the far UVC light sources (3) emit the far UVC light, are substantially horizontal and perpendicular to the walls (2), from which the far UVC light is emitted.

4. The UV booth (1) according to any of the preceding claims, wherein the far UVC light sources (3) are distributed evenly across the surfaces of the walls (2) facing towards the centre of the UV booth (1), for instance by being arranged in arrays of one or more horizontal rows and one or more vertical columns.

5. The UV booth (1) according to any of the preceding claims, further comprising a plurality of far UVC light sources (4) arranged along the bottom of one or more of the walls (2) to emit far UVC light in one or more partly upward directions towards the centre of the UV booth (1).

6. The UV booth (1) according to any of the preceding claims, further comprising a plurality of far UVC light sources arranged along the top of one or more of the walls (2) to emit far UVC light in one or more partly downward directions towards the centre of the UV booth (1).

7. The UV booth (1) according to any of the preceding claims further comprising a plurality of far UVC light sources (5) arranged along a vertical edge of one or more of the walls (2) to emit far UVC light in one or more directions towards the centre of the UV booth (1), which directions are substantially horizontal but not perpendicular to the walls (2), from which the far UVC light is emitted.

8. The UV booth (1) according to any of the preceding claims, wherein the number of far UVC light sources (3, 4, 5) is larger than 20, preferably larger than 50, most preferably larger than 100.

9. The UV booth (1) according to any of the preceding claims, wherein one or more sides of the three-dimensional space delimited by the walls (2), which are not covered by a wall (2), are provided with doors (13), such as glass doors.

10. The UV booth (1) according to claim 9, wherein the doors (13) are coated with a UVC-reflecting material on the side facing towards the centre of the UV booth (1) when the doors (13) are closed.

11. The UV booth (1) according to any of the preceding claims, wherein one or more touchless buttons (9, 10) are arranged within the UV booth (1) for allowing the gowned worker (6) to start and stop a decontamination cycle.

12. The UV booth (1) according to any of the preceding claims, wherein the wavelengths of the emitted far UVC light is in the range 210-230 nm, preferably in the range 220-225 nm, such as 222 nm.

13. The UV booth (1) according to any of the preceding claims, wherein the UV booth (1) comprises an electronic control system, which is programmed to automatically stop a decontamination cycle, when a predefined dosage of far UVC light has been delivered.

14. The UV booth (1) according to any of the preceding claims, wherein the UV booth (1) is dimensioned, constructed and controlled in such a way that the duration of a contamination cycle is less than two minutes, preferably less than one minute, such as 30 seconds.

15. Use of a UV booth (1) according to any of the preceding claims for decontamination of a gowned worker (6) wearing personal protective equipment, such as a gown, googles, a facemask and/or gloves.
